# FASCICULE DE BREVET EUROPEEN

(11) **EP 1 499 280 B1**
(45) Date de publication et mention de la délivrance du brevet: **21.07.2010**
(21) Numéro de dépôt: 03746846.9
(22) Date de dépôt: 17.04.2003
(51) Int. Cl.: A61K 8/46, A61K 8/73, A61Q 5/12, A61Q 5/02

(54) **UTILISATION DE LA BETA-CYCLODEXTRINE AVES DES TENSIOACTIFS EN TANT QU'AGENT NACRANT ET COMPOSITIONS NACREES**
VERWENDUNG VON BETA-CYCLODEXTRIN MIT TENSIDEN ALS PERLMUTTSCHIMMER-MITTEL
USE OF BETA-CYCLODEXTRINE WITH SURFATANTS AS A PEARLY-LUSTRING AGENT

(30) Priorité: 22.04.2002 FR 0205004
(43) Date de publication de la demande: 26.01.2005
(73) Titulaire: L'Oréal, 75008 Paris (FR)
(72) Inventeur: MÜLLER, Rainer, D-76344 Leopoldshafen (DE)
(74) Mandataire: Dossmann, Gérard
(86) Numéro de dépôt international: PCT/FR2003/001243
(87) Numéro de publication internationale: WO 2003/088934

(56) Documents cités:
- EP-A- 0 246 090
- US-A- 4 678 598
- US-A- 6 025 510
- PATENT ABSTRACTS OF JAPAN vol. 016, no. 283 (C-0955), 24 juin 1992 (1992-06-24) & JP 04 074107 A (SUNSTAR INC), 9 mars 1992 (1992-03-09)
- PATENT ABSTRACTS OF JAPAN vol. 011, no. 024 (C-399), 23 janvier 1987 (1987-01-23) & JP 61 197509 A (KAO CORP), 1 septembre 1986 (1986-09-01)

## Description

La présente invention a trait à l'utilisation d'une β-cyclodextrine en tant qu'agent nacrant, dans une composition notamment cosmétique comprenant dans un milieu aqueux physiologiquement acceptable au moins un tensioactif. L'invention a également pour objet des compositions nacrées comprenant dans un milieu aqueux physiologiquement acceptable au moins une β-cyclodextrine et au moins un tensioactif, elle a aussi pour objet des compositions nacrées comprenant dans un milieu aqueux physiologiquement acceptable au moins une β-cyclodextrine au moins un tensioactif et au moins un agent de conditionnement.

Par le terme "agent de nacrage" ou "agent nacrant", on entend un agent produisant un aspect ou un effet nacré, irisé, moiré ou métallisé.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà préconisé dans les compositions pour le lavage ou le soin des matières kératiniques telles que les cheveux l'utilisation d'agents de conditionnement, notamment insolubles, pour faciliter le démêlage des cheveux et pour leur communiquer douceur, brillance et souplesse.

On sait également que les produits en particulier cosmétiques ayant un aspect ou un effet irisé, moiré ou métallisé, sont largement appréciés par les consommateurs pour leur aspect esthétique et donnant une apparence de richesse au produit. Les agents qui apportent cet effet sont des agents de nacrage généralement sous forme de cristaux qui restent dispersés dans les compositions et qui réfléchissent la lumière.

Les dérivés d'esters à longue chaîne sont largement utilisés pour nacrer les compositions notamment cosmétiques. Cependant, ces dérivés peuvent présenter des problèmes de cristallisation qui entraînent une évolution de la viscosité des compositions au cours du temps.

On connaît également les dérivés d'éthers ou de thioéthers à longue chaîne tels que ceux décrits dans les demandes EP457688 et WO98/03155. Cependant, ces derniers agents opacifient les compositions sans apporter de nacrage aux compositions ou pas suffisamment.

Pour obtenir l'effet nacré, les compositions contenant les agents nacrants sont génaralement chauffés au dessus du point de fusion de l'agent nacrant puis refroidit pour le faire cristalliser.

Il a été constaté que ces agents nacrants, du fait de leur faible densité, présentaient souvent l'inconvénient de remonter à la surface du shampooing et d'y former une couche inesthétique pour le consommateur.

De plus ces composés à chaînes grasses présentent dans certains cas l'inconvénient d'apporter un toucher chargé aux cheveux, un manque de légèreté, de volume de la chevelure .

Il existe donc toujours un besoin pour de nouveaux agents nacrants qui ne présentent pas les inconvénient cités ci-dessus et qui permettent également l'utilisation de constituants critiques tels que les agents de conditionnement insolubles et en particulier les silicones.

La demanderesse a découvert, de façon surprenante, qu'il était possible de formuler des compositions notamment cosmétique pour le traitement des matières kératiniques, en particulier des shampooings présentant un aspect nacré tout en ayant les propriétés esthétiques et cosmétiques recherchées, en utilisant dans ces compositions une β-cyclodextrine et un tensioactif anionique.

La demanderesse a découvert, ce qui fait l'objet de l'invention, que l'utilisation de la β-cyclodextrine permettait de nacrer et/ou améliorer le nacrage des compositions notamment cosmétiques comprenant au moins un tensioactif anionique.

Le nacrage obtenu est très brillant. Par ailleurs, la préparation des compositions peut se faire à froid, ce qui est très avantageux.

L'invention a pour objet l'utilisation d'une β-cyclodextrine en tant que nouvel agent nacrant dans une composition cosmétique en particulier de lavage et/ou de conditionnement comprenant au moins un milieu aqueux physiologiquement acceptable et au moins un tensioactif, telle que définie à la revendication 5.

L'invention a également pour objet des compositions nacrées notamment cosmétiques telle que définie à la revendication 1 comprenant dans un milieu aqueux notamment physiologiquement acceptable au moins un tensioactif aniomique et une β-cyclodextrine ou l'un de ses dérivés, la β-cyclodextrine et le tensioactif étant présents à une concentration suffisante pour former un complexe insoluble dans la composition.

Les compositions présentent une très bonne homogénéité et une bonne stabilité du nacrage, ainsi qu'une viscosité satisfaisante pour l'application sur les matières kératiniques.

D'autres objets de l'invention apparaîtront à la lecture de la description et des exemples qui suivent.

Les cyclodextrines sont notamment des oligosaccharides de formule : 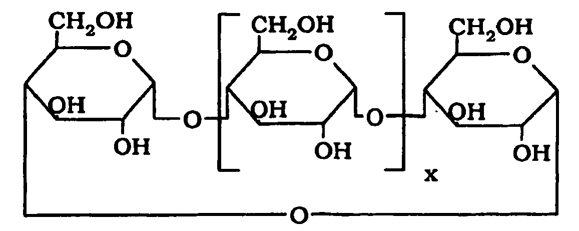 dans laquelle x est un nombre égal à 5 (β-cydodextrine).

On peut notamment utiliser une beta-cyclodextrine vendu par la société WACKER sous la dénomination CAVAMAX W7.

Selon l'invention, la β-cyclodextrine représente de 0,2% à 30 % en poids, de préférence de 1 % à 15 % en poids, et encore plus préférentiellement de 1,5 % à 10 % en poids, du poids total de la composition finale.

Les compositions de l'invention comprennent en outre au moins un tensioactif anionique qui est généralement présent en une quantité comprise entre 3 et 30% en poids.

La β-cyclodextrine et le tensioactif est présent une concentration efficace pour nacrer la composition et/ou pour former un complexe insoluble dans la composition entre la cyclodextrine et le tensioactif ou les tensioactifs.

Le rapport tensioactif/cyclodextrine peut varier de 0,01 à 300, de préférence de 0,1 à 100 et plus particulièrement de 0,3 à 25.

Les tensioactifs anioniques convenant à la mise en oeuvre de la présente invention de préférence sont solubles dans l'eau à température ambiante :

### (i) Tensioactif(s) anionique(s) :

Leur nature ne revêt pas, dans le cadre de la présente invention, de caractère véritablement critique.

Ainsi, à titre d'exemple de tensioactifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkyl sulfates, les alkyl éther sulfates, alkyl amido éther sulfates, alkyl aryl polyéther sulfates, monoglycérides sulfates ; les alkyl sulfonates, alkylphosphates, alkyl amido sulfonates, alkyl aryl sulfonates, α-oléfine-sulfonates, paraffine-sulfonates ; les alkyl sulfo succinates, les alkyl éther sulfosuccinates, les alkyl amide sulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyllactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides aikyl(C₆-C₂₄)aryl éther carboxyliques polyoxyalkylénés, les acides alkylamido(C₆-C₂₄) éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélanges avec :
- soit un agent tensioactif amphotère tel que les dérivés d'amine dénommés disodiumcocoamphodiacetate ou sodiumcocoamphopropionate commercialisés notamment par la société RHODIA CHIMIE sous la dénomination commerciale "MIRANOL^{®} C2M CONCNP" en solution aqueuse à 38 % de matière active ou sous la dénomination MIRANOL^{®} C32;
- soit un agent tensioactif amphotère tel que les alkylbétaïnes en particulier la cocobétaïne commercialisée sous la dénomination "DEHYTON^{®} AB 30" en solution aqueuse à 32 % de MA par la société COGNIS ou tel que les alkyl (C₈-C₂₀) amidoalkyl (C₁-C₆) bétaïnes en particulier la TEGOBETAINE^{®} F 50 commercialisée par la société GOLDSCHMIDT.

Les compositions cosmétiques selon l'invention contiennent également des agents de conditionnement des matières kératiniques.

L'invention a donc pour objet de compositions cosmétiques nacrées telle que définie à la revendication 1 en particulier de lavage et/ou de conditionnement comprenant dans un milieu aqueux physiologiquement acceptable au moins un tensioactif, au moins un agent de conditionnement, et au moins une β-cyclodextrine. Ces compositions, lorsqu'elles sont appliquées sur les cheveux; possèdent de bonnes propriétés de conditionnement des cheveux, c'est-à-dire que les cheveux traités sont lisses, se démêlent facilement, sont doux au toucher. Les cheveux ont un aspect naturel et non chargé.

Les compositions selon l'invention contenant des agents de conditionnement sont stables : en particulier, il ne se produit aucun relargage des agents de conditionnement ou d'épaississement incontrôlé de la composition au cours du temps. Les compositions présentent enfin une texture non filante et fondante. La mousse se rince facilement.

Un autre objet de l'invention est constitué par le procédé de lavage et/ou de conditionnement mettant en oeuvre de telles compositions.

L'agent de conditionnement est maintenu en suspension par le complexe formé entre le tensioactif et la cyclodextrine et non complexé par la cyclodextrine.

Les agents de conditionnement sont choisis parmi les poly-α-oléfines, les huiles fluorées, les cires fluorées, les gommes fluorées, les esters d'acides carboxyliques, les polymères cationiques, les silicones, les huiles minérales, végétales ou animales, les céramides, les pseudocéramides et leurs mélanges.

Les polyoléfines sont de préférence des poly-α-oléfines et en particulier :
- de type polybutène, hydrogéné ou non, et de préférence polyisobutène, hydrogéné ou non. ,

On utilisé de préférence les oligomères d'isobutylène de poids moléculaire inférieur à 1000 et leurs mélange avec des polyisobutylènes de poids moléculaire supérieur à 1000 et de préférence allant de 1000 à 15000.

A titre d'exemples de poly-α-oléfines utilisables dans le cadre de la présente invention, on peut plus particulièrement mentionner les polyisobutènes vendus sous le nom de PERMETHYL 99 A, 101 A , 102 A , 104 A (n=16) et 106 A (n=38) par la Société PRESPERSE Inc, ou bien encore les produits vendus sous le nom de ARLAMOL HD (n=3) par la Société ICI (n désignant le degré de polymérisation),
- de type polydécène, hydrogéné ou non.

De tels produits sont vendus par exemple sous les dénominations ETHYLFLO par la société ETHYL CORP., et d'ARLAMOL PAO par la société ICI.

Les huiles minérales pouvant être utilisées dans les compositions de l'invention sont choisies préférentiellement dans le groupe formé par :
- les hydrocarbures, tels que l'hexadécane et l'huile de paraffine ;

Comme huile végétale, on peut notamment mentionner l'huile d'amande douce, l'huile d'avocat, l'huile de ricin, l'huile d'olive, la cire de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huiles de maïs, l'huile de noisette, le beurre de karité, l'huile de palme, l'huile de noyau d'abricot, l'huile de calophyllum.

On peut également utiliser des huiles végétales transestérifiées, par exemple l'huile d'olive transestérifiée avec de l'hexanol, la cire de jojoba transestérifiée avec de l'éthanol.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression « polymère cationique » désigne tout polymère contenant des groupements cationiques et/ou des groupements ionisables en groupements cationiques.

Parmi tous les polymères cationiques susceptibles d'être utilisés dans le cadre de la présente invention, on préfère mettre en oeuvre les dérivés d'éther de cellulose quaternaires tels que les produits commercialisés sous la dénomination « JR 400 » par la société UNION CARBIDE CORPORATION, les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 100 », « MERQUAT 550 » et « MERQUAT S » par la société MERCK, les polysaccharides cationiques et plus particulièrement les gommes de guar modifiées par du chlorure de 2,3-époxypropyl triméthylammonium commercialisées par exemple sous la dénomination « JAGUAR C13S » par la société MEYHALL, les homopolymères et les copolymères éventuellement réticulés de sel de (méth)acryloyloxyéthyltriméthylammoniurn, vendus par la société ALLIED COLLOIDS en solution à 50% dans de l'huile minérale sous les dénominations commerciales SALCARE SC92 (copolymère réticulé du chlorure de méthacryloyloxyéthyltriméthylammonium et de l'acrylamide) et SALCARE SC95 (homopolymère réticulé du chlorure de méthacryloyloxyéthyl triméthylammonium).

On peut également utiliser les polymères qui sont constitués de motifs récurrents répondant à la formule : 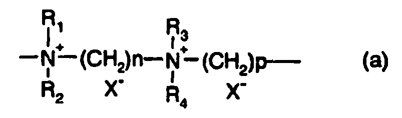 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.

Les silicones utilisables conformément à l'invention sont en particulier des polyorganosiloxanes insolubles dans la composition et peuvent se présenter sous forme d'huiles, de cires, de résines ou de gommes.

Les silicones insolubles dans l'eau sont insolubles dans l'eau à une concentration supérieure ou égale à 0,1% en poids dans l'eau à 25°C, c'est à dire qu'elles ne forment pas une solution isotrope transparente.

La viscosité des silicones est par exemple mesurée à 25°C selon la norme ASTM 445 Appendice C.

Les organopolysiloxanes sont définis plus en détail dans l'ouvrage de Walter NOLL "Chemistry and Technology of Silicones" (1968) Académie Press. Elles peuvent être volatiles ou non volatiles.

Lorsqu'elles sont volatiles, les silicones sont plus particulièrement choisies parmi celles possédant un point d'ébullition allant de 60° C à 260° C, et plus particulièrement encore parmi :
(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA CHIMIE, le décaméthylcyclopentasiloxane commercialisé sous le nom de "VOLATILE SILICONE 7158" par UNION CARBIDE, "SILBIONE 70045 V 5" par RHODIA CHIMIE, ainsi que leurs mélanges.
   On peut également citer les cyclocopolymères du type diméthylsiloxanes/ méthylakylsiloxane, tel que la "SILICONE VOLATILE FZ 3109" commercialisée par la société UNION CARBIDE, de structure chimique : 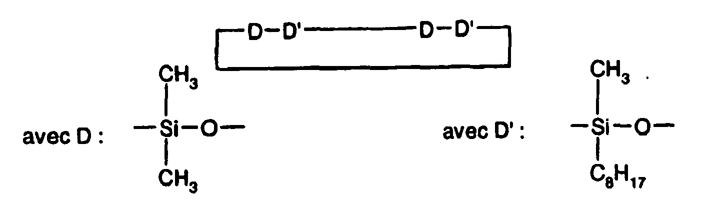 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toileries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s.

Parmi ces polyalkylsiloxanes, on peut citer à titre non limitatif les produits commerciaux suivants :
- les huiles de la série MIRASIL commercialisées par la société RHODIA CHIMIE, telles que par exemple l'huile MIRASIL DM 500 000 ;
- les huiles de la série 200 de la société DOW CORNING telles que plus particulièrement la DC200 de viscosité 60 000 cSt ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHODIA CHIMIE .

Dans cette classe de polyalkylsiloxanes, on peut également citer les produits commercialisés sous les dénominations "ABIL WAX 9800 et 9801" pair la société GOLDSCHMIDT qui sont des polyalkyl (C₁-C₂₀) siloxanes.

Les polyalkylarylsiloxanes sont particulièrement choisis parmi les polydiméthyl méthylphénylsiloxanes, les polydlméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
- les huiles MIRASIL DPDM de RHODIA CHIMIE;
- les huiles des séries RHODORSIL 70 633 et 763 de RHODIA CHIMIE ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phényiméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane
- les gommes polydiméthylsiloxanes/méthylvinylsiloxane,
- polydlméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à invention sont des mélanges tels que :
- les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q21401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :
R₂SiO_{2/2}, R₃SiO_{1/2}, RSiO_{3/2} est SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;

- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-8516334.
- des groupements acyloxyalkyle tels que par exemple les polyorganosiloxanes décrits dans le brevet US-A-4957732 ;
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701 E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255".
- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707, EP-A-640105 et WO 95/00578, EP-A-582 152 et WO 93/23009 et les brevets US 4,693,935, US 4,728,571 et US 4,972,037. Ces polymères sont de préférence anioniques ou non ioniques.

De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 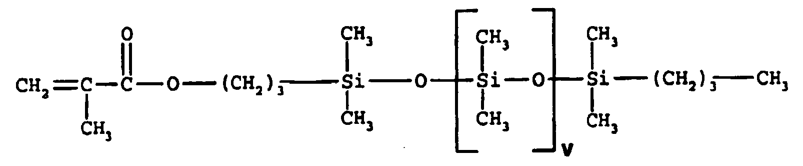 avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions.

Les polyorganosiloxanes particulièrement préférés conformément à l'invention sont :
- les silicones non volatiles choisies dans la famille des polyalkylsiloxanes à groupements terminaux triméthylsilyle telles que les huiles ayant une viscosité comprisse allant de 0,2 à 2,5 m²/s à 25° C telles que les huiles de la séries DC200 de DOW CORNING en particulier celle de viscosité 60 000 cSt, des séries MIRASIL DM et plus particulièrement l'huile MIRASIL DM 500 000 commercialisées par la société RHODIA CHIMIE ou l'huile de silicone AK 300.000 de la société WACKER, les polyalkylsiloxanes à groupements terminaux diméthylsilanol tels que les diméthiconol ou les polyalkylarylsiloxanes tels que l'huile MIRASIL DPDM commercialisée par la société RHODIA CHIMIE ;
- les polysiloxanes à groupements aminés tels que les amodiméthicones ou les triméthylsilylamodiméthicones ;

Selon la présente invention, les composés de type céramide sont notamment les céramides et/ou les glycocéramides et/ou les pseudocéramides et/ou les néocéramides, naturels ou synthétiques.

Des composés de type céramide sont par exemple décrits dans les demandes de brevet DE4424530, DE4424533, DE4402929, DE4420736, WO95/23807, WO94/07844, EP-A-0646572, WO95/16665, FR-2 673179, EP-A-0227994 et WO 94/07844, WO94/24097, WO94/10131 dont les enseignements sont ici inclus à titre de référence.

Des composés de type céramides particulièrement préférés selon l'invention sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-paimitoyt]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
- le (bis-(N-hydroxyéthyl N-cétyl) malonamide),
- le N-(2-hydroxyéthyl)-N-(3-cétyloxy-2-hydroxypropyl)amide d'acide cétylique
- le N-docosanoyl N-méthyl-D-glucamine
ou les mélanges de ces composés.

Selon l'invention, les agents conditionneurs représentent de 0,001 % à 10 % en poids, de préférence de 0,005 % à 5 % en poids, et encore plus préférentiellement de 0,01 % à 3 % en poids, du poids total de la composition finale.

Le milieu physiologiquement acceptable est de préférence constitué par de l'eau ou un mélange d'eau et de solvants cosmétiquement ou dermatologiquement acceptables tels que des monoalcools, des polyalcools, des éthers de glycol qui peuvent être utilisés seuls ou en mélange. L'eau erprésente de préférence de 30 à 98% en poids et de préférence de 50 à 98% en poids par rapport au poisd total de la composition.

On peut citer plus particulièrement les monoalcools tels que l'éthanol, l'isopropanol, les polyalcools tels que le diéthylèneglycol, la glycérine, les éthers de glycol, les alkyléthers de glycol ou de diéthylèneglycol.

La composition de l'invention peut également contenir au moins un additif choisi parmi les séquestrants, les adoucissants, les modificateurs de mousse, les colorants, d'autres agents nacrants, les agents hydratants, les agents antipelliculaires ou anti-séborrhéiques, d'autres agents de mise en suspension, les acides gras à chaînes linéaires ou ramifiées en C₁₆-C₄₀, les hydroxyacides, les électrolytes, les épaississants, les esters d'acides gras, les parfums, les conservateurs, les filtres solaires, les protéines, les vitamines et provitamines, les polymères, et tout autre additif classiquement utilisé dans le domaine cosmétique.

Ces additifs sont présents dans la composition selon l'invention dans des proportions pouvant aller de 0 à 40% en poids par rapport au poids total de la composition. La quantité précise de chaque additif est fonction de sa nature et est déterminée facilement par l'homme de l'art.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée. En particulier, pour avoir un effet nacré, la cyclodextrine ne doit généralement pas former de complexe avec l'agent de conditionnement et/ou les additifs présents dans la composition.

Les compositions selon l'invention peuvent se présenter sous forme de gel, de lait, de crème, de lotion plus ou moins épaissie ou de mousse.

Les compositions conformes à l'invention peuvent être utilisées pour le traitement des matières kératiniques telles que les cheveux, la peau, les cils, les sourcils, les ongles, les lèvres, le cuir chevelu et plus particulièrement les cheveux.

Les compositions peuvent également être utilisées pour le lavage et le nettoyage des matières kératiniques telles que les cheveux et la peau.

Les compositions selon l'invention sont utilisées généralement comme produits notamment pour le lavage, le soin, le conditionnement, le maintien de la coiffure ou la mise en forme des matières kératiniques telles que les cheveux.

Les compositions de invention peuvent plus particulièrement se présenter sous forme de shampooing, d'après-shampooing à rincer ou non, de compositions pour permanente, défrisage, coloration ou décoloration, ou encore sous forme de compositions à appliquer avant ou après une coloration, une décoloration, une permanente ou un défrisage ou encore entre les deux étapes d'une permanente ou d'un défrisage. De préférence, les compositions sont des compositions lavantes et moussantes pour les cheveux et/ou la peau.

Les compositions selon l'invention sont des compositions détergentes moussantes telles que des shampooings, des gels douche et des bains moussants. Selon l'invention, les compositions comprennent au moins un tensioactif détergent.

Le ou les tensioactifs détergents sont choisis, seuls ou en mélanges, au sein des tensioactifs anioniques décrits ci-dessus.

La quantité minimale tensioactif est celle juste suffisante pour conférer à la composition finale un pouvoir moussant et/ou détergent satisfaisant.

Ainsi, selon l'invention, le tensioactif détergent représente de 3 % à 30 % en poids, de préférence de 6 % à 25 % en poids, et encore plus préférentiellement de 8 % à 20 % en poids, du poids total de la composition finale.

Le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.

Les modifications de la méthode sont les suivantes :
La mesure se fait à la température de 22°C avec de l'eau osmosée. La concentration de la solution est de 2g/l. La hauteur de la chute est de 1 m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Lorsque la composition se présente sous la forme d'un après-shampooing éventuellement à rincer, elle contient avantageusement un tensioactif cationique, sa concentration allant généralement de 0,1 à 10% en poids et de préférence de 0,5 à 5% en poids par rapport au poids total de la composition.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

L'invention a encore pour objet un procédé de traitement cosmétique des matières kératiniques telles que les cheveux consistant à appliquer sur ceux-ci une composition telle que définie précédemment puis à effectuer éventuellement un rinçage à l'eau, après un éventuel temps de pause.

Les compositions sont préparées généralement en mélangeant la cyclodextrine et l'eau de la composition puis en additionnant le ou les tensioactifs.

L'invention va être maintenant plus complètement illustrée à l'aide des exemples suivants qui ne sauraient être considérés comme la limitant aux modes de réalisation décrits. Dans ce qui suit, MA signifie Matière Active.

### EXEMPLE 1

On a préparé trois shampooings de compositions suivantes :
La composition A est selon l'invention, la composition C est une composition comparative de l'art antérieur.

| - | A(invention) | B | C |
|---|---|---|---|
| - Lauryléther sulfate de sodium oxyéthyléné à 2,2 moles d'oxyde d'éthylène en solution aqueuse à 70% de MA | 15,5 gMA | 15,5 gMA | 15,5 gMA |
| - Cocoylbétaïne en solution aqueuse à 30% de MA | 2,9 gMA | 2,9 g MA | 2,9 g MA |
| - Beta-Cyclodextrine (CAVAMAX W7 de WACKER | 2,5 g | | |
| - Gamma-Cyclodextrine (CAVAMAX W8 de WACKER | | 2,5 g | |
| - Mélange d'alcool cétylique (50% en poids) et de hydroxystéaryl cétyl éther (50% en poids) | | | 2,5 g |
| - Diméthicone (MIRASIL DM 500000 de RHODIA CHIMIE) | 2,7 g | 2,7 g | 2,7 g |
| - Gomme de guar modifiée par du chlorure de 2,3-époxypropyl triméthyl ammonium (JAGUAR C13 S de RHODIA CHIMIE) | 0,05 g | 0,05 g | 0,05 g |
| - Carbomer (CARBOPOL 980 de NOVEON) | 0,2 g | 0,2 g | 0,2 g |
| - Cétostéaryl(50/50 en poids)sulfate de sodium | 0,75 g | 0,75 g | 0,75 g |
| - Propylèneglycol | 0,1 g | 0,1 g | 0,1 g |
| - Conservateurs, parfum | qs | qs | qs |
| - NaOH qs | pH 6,5-7 | pH 6,5-7 | pH 6,5-7 |
| - Eau déminéralisée qsp | 100 g | 100 g | 100 g |

### Compositions A et B :

On place dans une cuve de fabrication la quantité d'eau dont on a besoin, puis on introduit la gomme de guar modifiée, puis on ajoute le cétostéaryl sulfate de sodium et ensuite on ajoute le carbomer.

Après homogénéisation on introduit la cyclodextrine. Ensuite on ajoute le laurylsulfate de sodium à 2,2 moles d'oxyde d'éthylène. Après homogénéisation on introduit le diméthicone éventuellement prédispersé avec une partie des tensioactifs. Après formation de l'émulsion on ajoute les conservateurs, la bétaïne, le propylène glycol, le parfum et on ajuste le pH.

### Composition C :

On place dans une cuve de fabrication la quantité d'eau dont on a besoin, puis on introduit la gomme de guar modifiée, puis on ajoute le cétostéaryl sulfate de sodium et ensuite on ajoute le carbomer.

Après homogénéisation on ajoute le laurylsulfate de sodium à 2,2 moles d'oxyde d'éthylène puis la partie D. Après homogénéisation on introduit le diméthicone éventuellement prédispersé avec une partie des tensioactifs. Après formation de l'émulsion on ajoute les conservateurs, la bétaïne, le propylène glycol, le parfum et on ajuste le pH.

La Partie D est préparée en mélangeant à 70°C l'alcool cétylique et l'hydroxystéaryl cétyl éther avec du laurylsulfate de sodium à 2,2 moles d'oxyde d'éthylène en solution aqueuse dans un rapport en poids 2:1.

La composition selon l'invention a un effet blanc nacré plus important que celui de la composition Cet en particulier un effet nacrant très brillant.

Les cheveux lavés avec la composition A sont moins rêches et ont plus de volume que ceux traités avec la composition C.

## Revendications

1. Composition détergente moussante et nacrée notamment cosmétique **caractérisée en ce qu'**elle comprend dans un milieu aqueux au moins en agent tensioactif détergent, la β-cyclodextrine et au moins un agent de conditionnement, le tensioactif et la β-cyclodextrine étant présent dans des concentrations suffisantes pour former un complexe insoluble dans la composition, la β-cyclodextrine représentant de 0,2 à 30% en poids par rapport au poids total de la composition finale, le ou les agents tensioactifs détergents étant choisis parmi les tensioactifs anioniques et représentant de 3 à 30% en poids, du poids total de la composition finale, le ou les agents de conditionnement étant choisis parmi les poly-β-oléfines, les huiles fluorées, les cires fluorées, les gommes fluorées, les esters d'acides carboxyliques, les silicones, les polymères cationiques, les huiles minérales, végétales ou animales, les céramides, les pseudocéramides, et leurs mélanges, le ou les agents conditionneurs représentant de 0,001% à 10% en poids, du poids total de la composition.

2. Composition selon la revendication 1, **caractérisée en ce que** la β-cyclodextrine représente de 1% à 15% en poids, par rapport au poids total de la composition finale.

3. Composition selon l'une quelconque des revendications précédentes, **caractérisée par le fait qu'**elle se présente sous forme de gel, de lait, de crème, de lotion plus ou moins épaissie ou de mousse.

4. Composition selon l'une quelconque des revendications précédentes, **caractérisée par le fait que** la composition détergente moussante est choisie parmi les shampooings, les gels-douche et les bains moussants.

5. Utilisation d'une β-cyclodextrine en tant qu'agent nacrant dans une composition en particulier cosmétique comprenant au moins un milieu aqueux physiologiquement acceptable et au moins un tensioactif anionique détergent, la β-cyclodextrine représentant de 0,2 à 30% en poids par rapport au poids total de la composition finale, le ou les tensioactifs anioniques détergents représentant 3 à 30% en poids du poids total de la composition finale.

6. Procédé de traitement cosmétique des matières kératiniques, en particulier des cheveux, **caractérisé par le fait qu'**il consiste à appliquer sur lesdites matières une composition telle que définie selon l'une quelconque des revendications 1 à 4 puis à effectuer éventuellement un rinçage à l'eau.

## Claims

1. Foaming pearlescent, in particular cosmetic, detergent composition, **characterized in that** it comprises, in an aqueous medium, at least one detergent surfactant, β-cyclodextrin and at least one conditioning agent, the surfactant and the β-cyclodextrin being present in sufficient concentrations to form an insoluble complex in the composition, the β-cyclodextrin representing from 0.2% to 30% by weight relative to the total weight of the final composition, the detergent surfactant(s) being chosen from anionic surfactants and representing from 3% to 30% by weight of the total weight of the final composition, the conditioning agent(s) being chosen from poly-α-olefins, fluorinated oils, fluorinated waxes, fluorinated gums, carboxylic acid esters, silicones, cationic polymers, mineral, plant or animal oils, ceramides, pseudoceramides, and mixtures thereof, the conditioning agent(s) representing from 0.001% to 10% by weight of the total weight of the composition.

2. Composition according to Claim 1, **characterized in that** the β-cyclodextrin represents from 1% to 15% by weight relative to the total weight of the final composition.

3. Composition according to any one of the preceding claims, **characterized in that** it is provided in the form of a gel, a milk, a cream, a more or less thickened lotion or a mousse.

4. Composition according to any one of the preceding claims, **characterized in that** the foaming detergent composition is chosen from shampoos, shower gels and bubble baths.

5. Use of a β-cyclodextrin as a pearlescent agent in a composition, in particular a cosmetic composition, comprising at least one physiologically acceptable aqueous medium and at least one detergent anionic surfactant, the β-cyclodextrin representing from 0.2 to 30% by weight relative to the total weight of the final composition, the detergent anionic surfactant(s) representing from 3 to 30% by weight of the total weight of the final composition.

6. Method for the cosmetic treatment of keratinous materials, in particular hair, **characterized in that** it consists in applying to said materials a composition as defined according to any one of claims 1 to 4, and then in optionally rinsing with water.

## Patentansprüche

1. Schaumbildende und perlmuttschimmernde, reinigende Zusammensetzung und insbesondere kosmetische Zusammensetzung, **dadurch gekennzeichnet, dass** sie in einem wässrigen Medium mindestens einen reinigenden grenzflächenaktiven Stoff, β-Cyclodextrin und mindestens ein Konditioniermittel enthält, wobei der grenzflächenaktive Stoff und das β-Cyclodextrin in Konzentrationen vorliegen, die zur Bildung eines in der Zusammensetzung unlöslichen Komplexes ausreichend sind, wobei das β-Cyclodextrin 0,2 bis 30 Gew.-%, bezogen auf das Gesamtgewicht der fertigen Zusammensetzung, ausmacht, wobei der oder die reinigenden grenzflächenaktiven Stoffe unter den anionischen grenzflächenaktiven Stoffen ausgewählt sind und 3 bis 30 Gew.-% des Gesamtgewichts der fertigen Zusammensetzung ausmachen, wobei das oder die Konditioniermittel unter den Poly-αolefinen, fluorierten Ölen, fluorierten Wachsen, fluorierten Gummis, Carbonsäureestern, Siliconen, kationischen Polymeren, Mineralölen, pflanzlichen Ölen oder tierischen Ölen, Ceramiden, Pseudoceramiden und deren Gemischen ausgewählt sind und wobei das oder die Konditioniermittel 0,001 bis 10 Gew.-% des Gesamtgewichts der Zusammensetzung ausmachen.

2. Zusammensetzung nach Anspruch 1, **dadurch gekennzeichnet, dass** das β-Cyclodextrin 1 bis 15 Gew.-%, bezogen auf das Gesamtgewicht der fertigen Zusammensetzung, ausmacht.

3. Zusammensetzung nach einem der vorhergehenden Ansprüche, **dadurch gekennzeichnet, dass** sie als Gel, Milch, Creme, mehr oder weniger dickflüssige Lotion oder Schaum vorliegt.

4. Zusammensetzung nach einem der vorhergehenden Ansprüche, **dadurch gekennzeichnet, dass** die schaumbildende reinigende Zusammensetzung unter den Haarwaschmitteln, Duschgelen und Schaumbädern ausgewählt ist.

5. Verwendung eines β-Cyclodextrins als Perlglanzstoff in einer Zusammensetzung und insbesondere einer kosmetischen Zusammensetzung, die zumindest ein physiologisch akzeptables wässriges Medium und mindestens einen anionischen reinigenden grenzflächenaktiven Stoff enthält, wobei das β-Cyclodextrin 0,2 bis 30 Gew.-%, bezogen auf das Gesamtgewicht der fertigen Zusammensetzung, ausmacht und der oder die reinigenden anionischen grenzflächenaktiven Stoffe 3 bis 30 Gew.-% des Gesamtgewichts der fertigen Zusammensetzung ausmachen.

6. Verfahren zur kosmetischen Behandlung von Keratinsubstanzen und insbesondere Haaren, **dadurch gekennzeichnet, dass** es darin besteht, eine Zusammensetzung, wie sie in einem der Ansprüche 1 bis 4 definiert ist, auf die Keratinsubstanzen aufzutragen und dann gegebenenfalls mit Wasser zu spülen.
